# FASCICULE DE BREVET EUROPEEN

(11) **EP 1 284 963 B1**
(45) Date de publication et mention de la délivrance du brevet: **09.05.2012**
(21) Numéro de dépôt: 01938319.9
(22) Date de dépôt: 23.05.2001
(51) Int. Cl.: C07D 209/14, A61K 31/4045, A61P 5/18, C07D 209/42

(54) **COMPOSES POSSEDANT UNE ACTIVITE CALCIMIMETIQUE**
VERBINDUNGEN MIT CALCIMIMETISCHER WIRKUNG
NOVEL CALCIUM RECEPTOR ACTIVE MOLECULES AND METHOD FOR PREPARING SAME

(30) Priorité: 24.05.2000 FR 0006619
(43) Date de publication de la demande: 26.02.2003
(73) Titulaire: CENTRE NATIONAL DE LA RECHERCHE SCIENTIFIQUE (CNRS), 75794 Paris Cedex 16 (FR)
(72) Inventeur: RUAT, Martial, F-92340 Bourg la Reine (FR); DODD, Robert, F-75009 Paris (FR); FAURE, Hélène, Véronique, F-91190 Gif-sur-Yvette (FR); DAUBAN, Philippe, Marcel, F-91700 Saint-Geneviève-des-Bois (FR); KESSLER, Albane, F-75015 Paris (FR); POTIER, Pierre, Jean-Paul, F-75007 Paris (FR)
(74) Mandataire: Warcoin, Jacques
(86) Numéro de dépôt international: PCT/FR2001/001599
(87) Numéro de publication internationale: WO 2001/090069

(56) Documents cités:
- WO-A-00/21910
- WO-A-00/32578
- WO-A-00/35864
- WO-A-93/04373
- WO-A-96/12697
- US-A- 3 906 099
- CHEMICAL ABSTRACTS, vol. 125, no. 21, 18 novembre 1996 (1996-11-18) Columbus, Ohio, US; abstract no. 275909y, KOJIMA, KOICHI ET AL.: "Preparation of hexahydropyrazinoquinoline derivatives exhibiting excellent serotonin 5-HT1a receptor agonist effects." XP002161408 -& DATABASE CHEMICAL ABSTRACTS [en ligne] CA 125:275909, XP002161453 & WO 96 23789 A (SANKYO CO., LTD.) 8 août 1996 (1996-08-08)
- CHEMICAL ABSTRACTS, vol. 129, no. 18, 2 novembre 1998 (1998-11-02) Columbus, Ohio, US; abstract no. 225222f, CHERQAOUI, DRISS ET AL.: "Structure-selectivity relationships of MAO inhibitors using neural networks" XP002161448 & ACH- MODELS CHEM., vol. 135, no. 1-2, - 1998 pages 79-91, -& DATABASE CHEMICAL ABSTRACTS [en ligne] 129:225222, XP002161454
- CHEMICAL ABSTRACTS, vol. 107, no. 3, 20 juillet 1987 (1987-07-20) Columbus, Ohio, US; abstract no. 23198n, ALVAREZ GAVELA, L. ET AL.: "Preparation of acetylenic and allenic derivatives of 2-indolylmethylamines and preliminary results of their study as selective inhibitors for the monoamine oxidases A and B." XP002161449 & AN. QUIM., SER C, vol. 82, no. 2, - 1982 pages 129-134, -& DATABASE CHEMICAL ABSTRACTS [en ligne] CA 107:23198, XP002161455
- CHEMICAL ABSTRACTS, vol. 106, no. 5, 2 février 1987 (1987-02-02) Columbus, Ohio, US; abstract no. 32831v, FERNANDEZ ALVAREZ, ELDIBERTO ET AL.: "2-((Alkynylamino)methyl)indoles." XP002161450 & ES 542 696 A (CONSEJO SUPERIOR DE INVESTIGACIONES CIENTIFICAS) 16 avril 1985 (1985-04-16) -& DATABASE CHEMICAL ABSTRACTS [en ligne] CA 106:32831, XP002161456
- CHEMICAL ABSTRACTS, vol. 52, no. 13, 10 juillet 1958 (1958-07-10) Columbus, Ohio, US; abstract no. 11039d, TAMAS NOGRADI: "Rauwolfia alkaloid models. II. 2-Substituted indole, tetrahydrocarboline, and hexahydroyohimban derivatives." XP002161451 & MONATSH. CHEM., vol. 88, - 1958 pages 1087-1094, -& DATABASE CHEMICAL ABSTRACTS [en ligne] 52:11039, XP002161457
- CHEMICAL ABSTRACTS, vol. 115, no. 5, 5 août 1991 (1991-08-05) Columbus, Ohio, US; abstract no. 49399u, FERNANDEZ ALVAREZ, ELDIBERTO ET AL.: "Preparation of acetylenic and allenic derivatives of 2-aminomethyl-1-methylindoles as momoamine oxidase (MAO) inhibitors." XP002161464 & ES 2 010 406 A (CONSEJO SUPERIOR DE INVESTIGACIONES CIENTIFICAS) 1 novembre 1989 (1989-11-01) -& DATABASE CHEMICAL ABSTRACTS [en ligne] CA 115:49399, XP002161465
- CHEMICAL ABSTRACTS, vol. 89, no. 3, 17 juillet 1978 (1978-07-17) Columbus, Ohio, US; abstract no. 21597t, VASIL'EVA V. F. ET AL.: "Substituted 1,2,3,4-tetrahydroquinolines. V. N-Carboxamidines and guanidines. Synthesis and pharmacological properties." XP002161452 & KHIM.-FARM. ZH., vol. 12, no. 8, - 1978 pages 40-45, -& DATABASE CHEMICAL ABSTRACTS [en ligne] CA 89:21579, XP002161458

## Description

La présente invention concerne les composés ayant une activité calcimimétique. Elle concerne en particulier une nouvelle classe de composés aminés, leur préparation, les compositions pharmaceutiques les comprenant et leur utilisation comme modulateur de l'activité des récepteurs aux ions (Ca²⁺)ₑ et (Mg²⁺)ₑ ou CaSR pour Calcium Sensing Receptor et comme médicament destiné de préférence au traitement des maladies ou des désordres physiologiques faisant intervenir la modulation de l'activité des CaSR.
L'activité calcimimétique correspond à la capacité de produire ou d'induire des réponses biologiques observées par les variations de la concentration des ions calcium extracellulaires (Ca²⁺)ₑ et des ions magnésium extracellulaires (Mg²⁺)ₑ.

Les ions (Ca²⁺)ₑ et (Mg²⁺)ₑ jouent un rôle majeur dans l'organisme car ils régulent l'homéostasie calcique dont dépendent les fonctions vitales de l'organisme. Ainsi, les hypercalcémies, c'est à dire des états ou les ions (Ca²⁺)ₑ sont au-dessus du seuil moyen, ont une incidence majeure sur de nombreuses fonctions telles que les fonctions cardiaques, rénales ou intestinales. Elles affectent profondément le système nerveux central (Chattopadhyay et al, Endocr. Review, 1998).

Les CaSR sont des protéines sensibles aux ions (Ca²⁺)ₑ et (Mg²⁺)ₑ et sont présentes dans les glandes parathyroïdiennes, thyroïdiennes, le rein, l'intestin, les poumons, les cellules osseuses, le cerveau, la moelle épinière, l'hypophyse, l'estomac, les kératinocytes (Brown et al, Nature, 1993 ; Ruat et al, Proc. Natl. Acad Sci., USA, 1995 ; Brown et al, Am. Rev. Med., 1998). Ces protéines sont codées par un seul gène isolé dans différentes espèces animales. Elles appartiennent à la famille des récepteurs couplés aux protéines G à sept domaines transmembranaires, et présentent des homologies de structure avec les récepteurs métabotropiques du glutamate, des récepteurs GABA_{B}, des récepteurs hypothétiques aux phéromones et du goût. Des mutations activatrices ou inhibitrices du gène chez l'homme sont responsables de maladies génétiques extrêmement graves qui provoquent des hypocalcémies ou des hypercalcémies (Pollack et al, Cell, 1993 ; Pollack et al, Nature Genetic, 1994 ; Brown et al, Ann. Rev Med, 1998). Les fonctions liées à l'expression de ces protéines dans les tissus ne sont pas encore toutes connues et font l'objet d'une très grande activité de recherche, particulièrement en ce qui concerne les CaSR présents dans les glandes parathyroïdiennes, thyroïdiennes, le rein, l'intestin, la moelle épinière, le cerveau et les cellules osseuses.

Dans la glande parathyroïdienne, les CaSR modulent la sécrétion de l'hormone parathyroïdienne (PTH) qui est le principal régulateur de l'homéostasie calcique: l'augmentation des ions (Ca²⁺)ₑ dans le sérum va activer les CaSR présents sur les cellules de la glande parathyroïdienne et diminuer la sécrétion de l'hormone PTH.

L'ADN complémentaire codant pour le CaSR de rat a été isolé à partir d'une banque ADNc de striatum de rat (Ruat et al, Proc. Natl. Acad Sci., 1995). Ce récepteur est identique au niveau de sa séquence en acides aminés à celui exprimé dans les autres tissus. Des cellules ovariennes de hamster chinois (CHO) transfectées exprimant le CaSR de rat (CHO(CaSR)) ont été caractérisées et les signaux chimiques (seconds messagers) induits par l'activation de ce récepteur ont été analysés. Ainsi, un test biochimique permettant de mesurer l'accumulation d'inositols phosphates tritiés [³H]IP en réponse à l'activation du récepteur a été mis au point (Ruat et al, J. Biol. Chem., 1996 ; Ferry et al, Biochem. Biophys Res. Commun., 1997).

Il a été montré que les ions Ca²⁺, Mg²⁺, mais aussi Ba²⁺ dans des gammes de concentrations millimolaires stimulent les CaSR. L'activation des CaSR pourrait être induite dans le cerveau par les peptides β-amyloides, qui sont impliqués dans des maladies neurodégénératives telles que la maladie d'Alzheimer (Ye et al, J. Neurosci. Res., 1997).

La perturbation de l'activité des CaSR est associée à des désordres biologiques tels que les hyperparathyroïdies primaires et secondaires, l'ostéoporose, les maladies cardio-vasculaires, gastro-intestinales, endocrines, neurodégénératives, ou encore certains cancers où les ions (Ca²⁺)ₑ sont anormalement élevés.

L'hyperparathyroïdie secondaire est observée lors d'insuffisance rénale chronique et se caractérise par une hyperplasie des glandes parathyroïdiennes et une augmentation de la PTH circulante. L'insuffisance rénale est aussi accompagnée d'ostéodystrophie rénale qui se caractérise par des désordres osseux avec un fort ou un faible renouvellement de la masse osseuse (ostéitis fibrosa, osteomalacia).

L'ostéoporose est une maladie multifactorielle qui dépend notamment de l'âge et du sexe. Si les femmes ménopausées sont très fortement touchées, l'ostéoporose s'avère de plus en plus un problème chez l'homme âgé, et il n'existe pas pour l'instant de traitements vraiment satisfaisants. Son coût social pourrait s'alourdir encore dans les prochaines années, particulièrement dans notre société européenne ou la durée de vie s'allonge. L'ostéoporose est actuellement traitée par les oestrogènes, la calcitonine ou les biphosphonates qui préviennent la résorption osseuse sans stimuler une nouvelle croissance osseuse. Des données plus récentes démontrent que des augmentations intermittentes de la PTH ou de ses dérivés, sont efficaces dans le traitement de l'ostéoporose et permettent de remodeler l'os en stimulant la formation osseuse (Whitfield et al, 1999). Cette nouvelle voie thérapeutique du traitement de l'ostéoporose apparaît très intéressante bien que des problèmes majeurs soient liés à l'utilisation de l'hormone PTH tels que la voie d'injection, mais aussi l'apparition de tumeurs observées récemment durant des essais cliniques chez l'homme. La sécrétion intermittente de PTH endogène peut être obtenue par le blocage du récepteur au calcium. Le blocage de la sécrétion de PTH par les agonistes du CaSR peut être suivie par une augmentation rapide de la PTH (effet rebond), qui est alors bénéfique dans le traitement de l'ostéoporose.

La recherche de ligands sélectifs pour les CaSR a conduit la société NPS à développer deux grands types de famille de composés organiques, à savoir les polyamines telles que le NPS 019 (3), et les arylalkylamines dont le représentant le plus connu à ce jour est le NPS R-568 (2).

Le composé NPS R-568 (2), ligand allostérique du CaSR, appartient à la première famille de molécules organiques de petite taille (M < 600), interagissant avec ce récepteur. Cette arylalkylamine a été développée à partir de la structure de la Fendiline (1), un puissant activateur du CaSR de la glande parathyroïdienne.

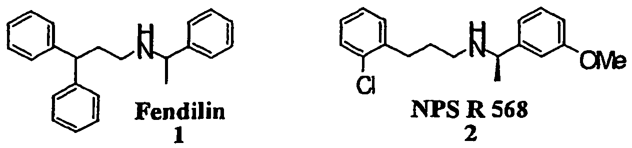

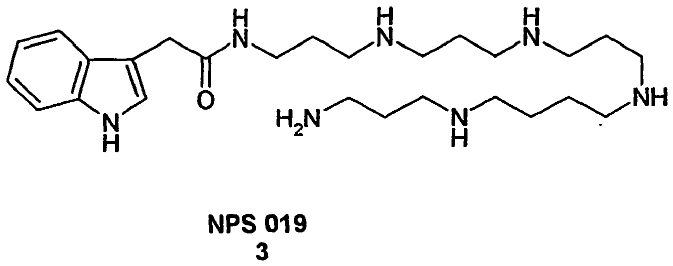

L'agent NPS-R-568 réduit ou élimine l'ostéitis fibrosa chez le rat (Wada et al, Kidney International, 1998) et réduit les concentrations de PTH chez des patients (hommes) souffrant d'insuffisance rénale chronique (Antansen et al, Kidney International, 1998). Ce composé a été utilisé par voie orale avec succès pour abaisser les concentrations de PTH et des ions Ca²⁺ libres sériques chez la femme ménopausée souffrant d'hyperparathyroïdie primaire (Silverberg et al, New Engl. J. Med, 1997). Dans une autre étude, le composé NPS-R-568 a permis de réduire entre 20-50 % la prolifération cellulaire observée dans la glande parathyroïdienne chez un modèle de rat reproduisant l'insuffisance rénale chronique (Wada et al, J. Clin. Invest., 1997). Ces études démontrent qu'un composé calcimimétique, actif vis-à-vis du récepteur au calcium présent sur la glande parathyroïde, peut-être considéré comme un outil thérapeutique intéressant pour traiter certaines formes d'hyperparathyroïdies primaires et secondaires.

Durant des essais cliniques, (Phase I - II) la société NPS Pharmaceutical a observé une bio-disponibilité faible du composé NPS-R-568 ainsi que des effets cliniques variables suivant les individus qui pourraient provenir de polymorphisme du gène codant pour le CaSR chez l'homme (Nemeth et al, Trends Endoc. Metab, 1999). De plus, lors d'essais expérimentaux chez le rat, le composé NPS R-467, un composé de structure voisine du NPS R-568, s'est avéré plus sélectif vis à vis des récepteurs de la parathyroïde comparativement à ceux de la glande thyroïde. Cette sélectivité peut s'expliquer par des différences liées aux tissus ce qui suggère que des molécules calcimimétiques spécifiques d'un tissu peuvent être synthétisées et avoir des importances cliniques considérables.

Parallèlement, il a été récemment rapporté la préparation et l'activité calcimimétique d'arylalkyl-1,2-diamines possédant la structure générale décrite ci-dessous (4), et parmi lesquelles le composé PHD 321 (5) constitue un des produits les plus actifs.

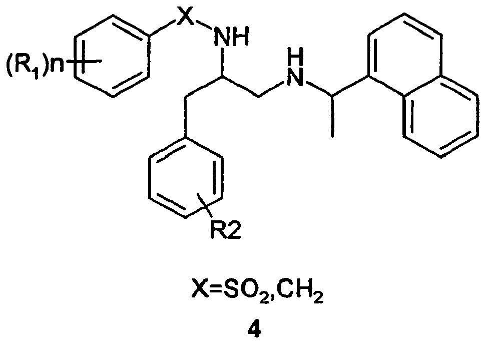

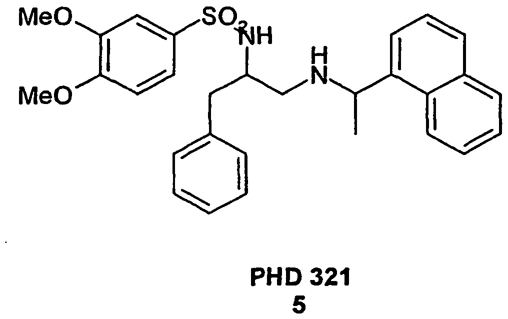

L'absence totale de molécules calcimimétiques en clinique, et les problèmes rencontrés en phase I-II pour les calcimimétiques de première génération soulignent la nécessité de trouver de nouvelles molécules calcimimétiques.

Dans cette invention, les molécules synthétisées présentent des avantages par rapport au composé NPS-R-568 et au composé NPS 019 car leur structure fait apparaître plusieurs sites d'interaction avec le CaSR ou avec son système de transduction.

Les composés selon l'invention sont représentés par la formule générale II suivante : 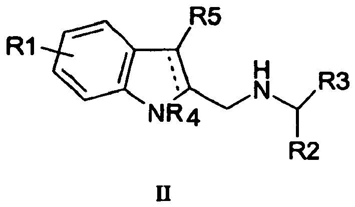 dans laquelle :
les groupes R1 et R5 sont tels que définis dans la revendication 1,
le groupe R2 représente un atome d'hydrogène ou un groupe alkyle en C₁-C₆
le groupe R3 représente un groupe aryle
et le groupe R4 représente un atome d'hydrogène, un groupe alkyle, aryle, aralkyle, alkylsulfonamide, arylsulfonamide ou aralkylsulfonamide et leurs sels avec un acide pharmaceutiquement acceptables à l'exception des composés dont la formule est indiquée dans la revendication 1.

De façon avantageuse, le groupe R3 représente un groupe phényle ou naphtyle substitué ou non.
Des exemples de composés avantageux selon l'invention sont ceux ayant pour formule III : 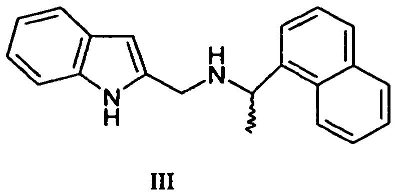 ou IV :

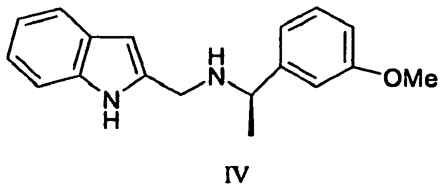

Par le terme « acide pharmaceutiquement acceptable » on entend au sens de la présente invention tout acide non toxique, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoique, pantothénique, phosphorique, succinique, sulfurique, tartarique et paratoluènesulfonique. L'acide chlorhydrique est particulièrement préféré.

Par le terme « groupe alkyle», on entend au sens de la présente invention tout groupe alkyle en C₁-C₆ linéaire ou ramifié, substitué ou non, en particulier, le groupe méthyle.

Par le terme « groupe alkoxy », on entend au sens de la présente invention tout groupe alkoxy de 1 à 6 atomes de carbones, linéaires ou ramifiés substitué ou non, en particulier, le groupe OCH₃.

Par le terme « groupe aryle », on entend au sens de la présente invention un ou plusieurs cycles aromatiques ayant 5 à 8 atomes de carbones, pouvant être accolés ou fusionnés, substitués ou non. En particulier, les groupes aryles peuvent être des groupes phényle ou naphthyle et les substituants des atomes d'halogène, des groupes alkoxy tels que définis ci-dessus, des groupes alkyle tels que définis ci-dessus ou le groupe nitro.

Par le terme « groupe aralkyle », on entend au sens de la présente invention tout groupe aryle tel que défini ci-dessus, lié par l'intermédiaire d'un groupe alkyle tel que défini ci-dessus. En particulier un groupe aralkyle est un groupe benzyle.

Par le terme « groupe alkylsulfonamide », on entend au sens de la présente invention tout groupe alkyle tel que défini ci-dessus, lié par l'intermédiaire d'un groupe sulfonamide.

Par le terme « groupe arylsulfonamide », on entend au sens de la présente invention tout groupe aryle tel que défini ci-dessus, lié par l'intermédiaire d'un groupe sulfonamide. En particulier, un groupe arylsulfonamide est le groupe

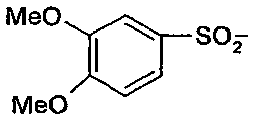

Par le terme « groupe aralkylsulfonamide, on entend au sens de la présente invention tout groupe aralkyle tel que défini ci-dessus, lié par l'intermédiaire d'un groupe sulfonamide.

Les composés selon l'invention possèdent tous un centre d'asymétrie et peuvent donc exister sous forme d'isomères optiques. La présente invention comprend aussi bien ces isomères soit séparément soit en tant que mélange.

La présente invention concerne également le mode de préparation de ces composés.

Ce procédé comprend en particulier les étapes de
a) Transformation de l'acide carboxylique de formule générale VIII suivante : 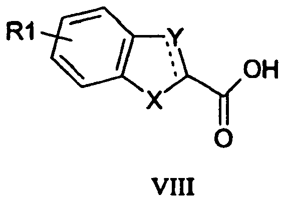 dans laquelle:
   le groupe X représente un groupe -NR4,
   le groupe Y représente un groupe -CR5, ou -CHR5,
   les groupes R1, et R5 sont tels que définis dans la revendication 6
   et le groupe R4 représente un atome d'hydrogène, un groupe alkyle, aryle aralkyle, alkylsulfonamide, arylsulfonamide, aralkylsulfonamide en chlorure d'acyle de formule générale VI suivante : 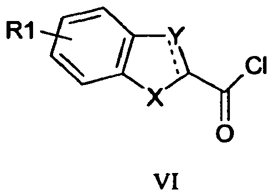
   dans laquelle :
   le groupe X représente un groupe -NR4,
   le groupe Y représente un groupe -CR5, ou -CHR5,
   les groupes R1; et R5 sont tels que définis dans la revendication 5
   et le groupe R4 représente un atome d'hydrogène, un groupe alkyle, aryle aralkyle, alkylsulfonamide, arylsulfonamide, aralkylsulfonamide, avantageusement par réaction avec un chlorure de thionyle.
b) Réaction entre le chlorure d'acyle de formule générale VI et une amine de formule générale VII suivante 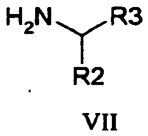 dans laquelle :
   le groupe R2 représente un atome d'hydrogène ou un groupe alkyle en C₁-C₆ et le groupe R3 représente un groupe aryle
   pour obtenir l'amide de formule générale V suivante : 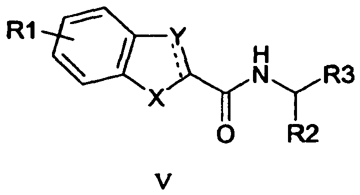 dans laquelle :
   le groupe X représente un groupe -NR4,
   le groupe Y représente un groupe -CR5,-ou-CHR5,
   les groupes R1,- et R5 sont tels que définis dans la revendication 4
   le groupe R2 représente un atome d'hydrogène ou un groupe alkyle, en C₁-C₆
   le groupe R3 représente un groupe aryle
   et le groupe R4 représente un atome d'hydrogène, un groupe alkyle, aryle aralkyle, alkylsulfonamide, arylsulfonamide ou aralkylsulfonamide.
c) Réduction de l'amide de formule générale V, avantageusement à l'aide de LiAlH₄, en composé de formule générale II.

Les acides carboxyliques de formule générale VIII et les amines de formule générale VII sont facilement disponibles dans le commerce.

La simplicité de cette synthèse et son très bon rendement permet d'introduire une grande variété de substituants R1, R2, R3, R4,- et R5 sur le composé de formule générale II.

L'utilisation dans le procédé de synthèse d'un isomère optiquement pur de l'amine de formule générale VII permet la synthèse d'un composé de formule générale II optiquement pur.

La présente invention concerne également les amides de formule générale V suivante: 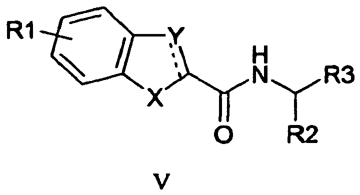 dans laquelle:
le groupe X représente un groupe -NR4,
le groupe Y représente un groupe
les groupes R1,- et R5 sont tels que définis à la revendication 7
le groupe R2 représente un atome d'hydrogène ou un groupe alkyle, en C₁-C₆
le groupe R3 représente un groupe aryle
et le groupe R4 représente un atome d'hydrogène, un groupe alkyle, aryle ou aralkyle, alkylsulfonamide, arylsulfonamide ou aralkylsulfonamide. et leur sels avec un acide pharmaceutiquement acceptable
   à l'exception des composés dont la formule est indiquée dans la revendication 7. Ces amides sont un intermédiaire réactionnel de la synthèse du composé de formule générale II.
   Les amides selon l'invention possèdent tous un centre d'asymétrie et peuvent donc exister sous forme d'isomères optiques. La présente invention comprend aussi bien ces isomères soit séparément soit en tant que mélange

La présente invention concerne également les compositions pharmaceutiques comprenant à titre de principe actif un des composés définis ci-dessus et un excipient approprié. Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriés comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

La présente invention concerne également l'utilisation des ces composés et des compositions pharmaceutiques les comprenant comme modulateur de l'activité du CaSR
Le CaSR peut se trouver dans la glande parathyroïde, la thyroïde, les cellules osseuses, l'estomac, le poumon, le rein, l'hypophyse, le cerveau, l'hypothalamus, les aires olfactives ou l'hippocampe.

Les composés selon la présente invention sont de préférence plus sélectifs dans leur utilisation vis à vis des récepteurs de la parathyroïde comparativement à ceux de la glande thyroïde.

Les composés selon l'invention et les compositions pharmaceutiques les comprenant peuvent être utilisés comme médicament, en particulier pour le traitement des maladies ou des désordres physiologiques liés à des perturbations de l'activité des CaSR. De façon encore plus particulière, ces maladies ou désordres physiologiques sont du type hyperparathyroïdies primaires ou secondaires, ostéoporose, maladies cardio-vasculaires, gastro-intestinales, endocrines, neurodégénératives ou certains cancers où les ions (Ca²⁺)ₑ sont anormalement élevés. L'hyperparathyroïdie secondaire est plus particulièrement observée lors d'insuffisance rénale chronique.

L'exemple de synthèse suivant de composés selon l'invention est donné à titre non limitatif et illustre l'invention.

### Synthèse du (R)-N-[1-(1-naphtyl)éthyl]indole-2-carboxamide

A une solution de l'acide indole-2-carboxylique (242 mg; 1,5 mmole) dans 10 ml de chloroforme sont successivement additionnés, à température ambiante sous argon, 7 équivalents de chlorure de thionyle (0,760 ml; 10,5 mmoles) et 0,020 ml de N,N-diméthylformamide (DMF). Après 1 nuit d'agitation à 30°C, le milieu est évaporé à sec pour donner le chlorure d'acide correspondant sous la forme d'un résidu huileux jaune. Ce dernier est dissous dans 10 ml de chloroforme puis 1,5 équivalent de triéthylamine (0,315 ml; 2,25 mmole) et 1,5 équivalent de (R)-1-(1-naphtyl)éthylamine (0,360 ml; 2,25 mmoles) sont ajoutés. La solution est agitée 2 heures à température ambiante avant d'être concentrée puis purifiée sur colonne de silice (éluant: heptane / acétate d'éthyle: 4 / 1). 340 mg (1,08 mmole; 72%) de l'amide sont isolés sous la forme d'un solide jaune.
*Spectrométrie de masse (ES)* : m/z: 315 (M+H]⁺

### Synthèse de la (R)-N-[(indol-2-yl)méthyl]-N-[1-(1-naphtyl)éthyl]amine.HCl (PHD 337-R)

A une solution d'hydrure mixte d'aluminium et de lithium LiAH₄ (60 mg; 1,6 mmole) dans 2 ml de tétrahydrofuranne (THF) distillé est additionnée, à température ambiante sous argon, une solution de chlorure d'aluminium AlCl₃ (213 mg; 1,6 mmole) dans 4 ml de THF distillé. La solution est agitée 45 minutes à température ambiante avant l'introduction par portions de l'amide (252 mg; 0,80 mmole). Le milieu réactionnel est alors chauffé à 60°C. Après 6 heures de réaction, 2 nouveaux équivalents de LiAIH₄ (60 mg; 1,6 mmole) sont ajoutés. La réaction est laissée la nuit à 60°C puis, après refroidissement, le milieu est hydrolysé par addition lente de THF 10% aqueux. Le mélange est ensuite traité par une solution de soude molaire avant d'être extrait 3 fois à l'éther éthylique. La phase organique est séchée sur sulfate de sodium, évaporée et purifiée sur colonne de silice (éluant: heptane / acétate d'éthyle: 60/ 40). 200 mg (0,66 mmole; 83%) d'une huile jaune sont isolés. L'amine libre est alors transformée en son chlorhydrate par traitement dans le chloroforme avec une solution saturée d'acide chlorhydrique dans le méthanol.
*Point de fusion* : 204 °C
*Spectrométrie de masse (ES) :* m/z: 301 [M+H]⁺
*Pouvoir rotatoire :* [α]²⁰_{D} = + 25,0° (c 0.4, CHCl₃)

### Activité sur des cellules transfectées exprimant le récepteur sensible aux ions(Ca²⁺)ₑ

L'activité calcimimétique des composés a été estimée en mesurant l'accumulation d'inositols phosphates tritiés induite par 10 µM de chacun des composés en présence de 2 mM de Ca²⁺ dans les cellules CHO(CaSR) (Ferry et al, Biochem Biophys Res Commun, 1997).

Cette activation a été comparée à celle induite par le composé NPS-R 568, un calcimimétique de référence et utilisé à une concentration de 10 µM (Tableau 1) (Ferry et al, Biochem Biophys Res Commun, 1997; Nemeth et al, Proc Natl Acad Sci USA, 1997).

Les composés PHD 337, PHD 337R, et PHD 356 utilisés à une concentration de 10 µM présentent une activité allant de 90 à 100 % de celle obtenue par 10 mM de Ca²⁺ comparable à celle du composé NPS-R-568 à la même concentration (Tableau 1). Il est à noter (Tableau 2) que les composés PHD 337, PHD 337R ou le composé PHD 356 à 1 µM présentent une activité supérieure à 50 % de celle du calcium (10 mM) alors que le composé PHD 363 est moins actif à cette même concentration suggérant un rôle important du groupe R1 sur le noyau phényle.

**Tableau 1: Accumulation d'inositols phosphates tritiés dans les cellules CHO(CaSR) induite par les composés PID/AK et le composé calcimimétique NPS R-568**

| Composé - (10 µM) | Formule brute - Masse molaire (point de fusion) | Structure | Accumulatior d'(³H)-IP % de la réponse 10mM Ca2⁺ ± E.S. |
|---|---|---|---|
| **NPS R-568** | C₁₈H₂₂ClNO.HCl 340,29 | 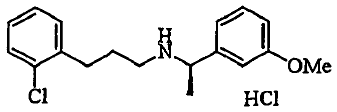 | 106 ± 15 |
| **PHD 337** | C₂ₗH₂₀N₂.2HCl 373,32 | 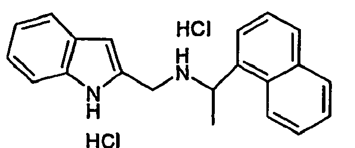 | 103 ± 6 |
| **PHD 337-R** | C₂₁H₂₀N₂.2HCl 373,32 | 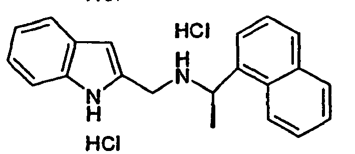 | 117 ± 5 |
| **PHD 337-S** | C₂₁H₂₀N₂.2HCl 373,32 | 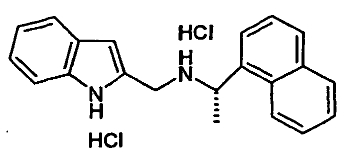 | 32 ± 5 |
| **PHD 356** | C₂₉H₂₀N₂O.2HCl 353,29 | 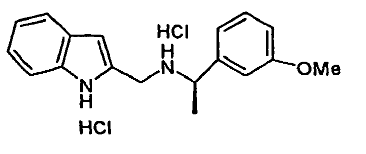 | 105 ± 15 |
| **PHD 359** | C₂₂H₂₂N₂O.2HCl 403,34 | 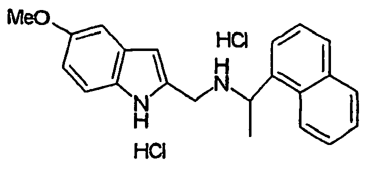 | 55 ± 5 |
| **PHD 361** | C₂₁H₁₉FN₂.2HCl 391,32 | 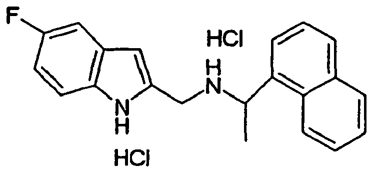 | 87 ± 7 |
| **PHD 363** | C₂₁H₁₉ClN₂.2HCl 407,77 | 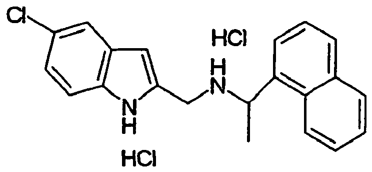 | 60 ± 1 |
| **PHD 368** | C₂₁H₂₂N₂.2HCl 375,34 | 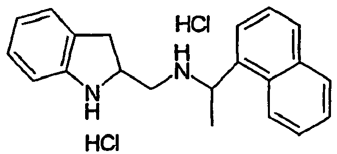 | 77 ± 1 |
| **PHD 338** | C₂₉H₂₈N₂O₄S.2HCl 537,08 | 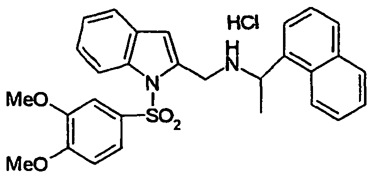 | 54 f 5 |
| **AK 43** | C₂₂H₂₂N₂.2HCl 350,89 | 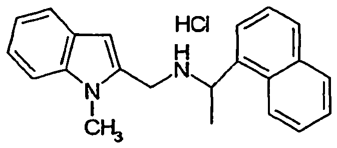 | 79 ± 13 |

L'activité calcimimétique des composés PHD/AK est comparée à celle d'un composé de référence, le NPS R-568 utilisé à la même concentration et dans les mêmes conditions expérimentales. Cette activité est exprimée en pourcentage de l'activité de 10 mM de Ca²⁺. Les moyennes ± erreurs standards moyennées de 2 à 5 manipulations indépendantes sont indiquées. Les expériences ont été réalisées en présence de 2 mM de Ca²⁺.

**Tableau 2: Accumulation d'inositols phosphates tritiés dans les cellules CHO(CaSR) induite par les composés PHD à 1 µM**

| Composé - (1 µM) | Formule brute - Masse molaire (point de fusion) | Structure | Accumulation d'(³H)-IP % de la réponse 10mM Ca²⁺ ± E.S. |
|---|---|---|---|
| **PHD 337** | C₂₁H₂₀N₂.2HCl 373,32 | 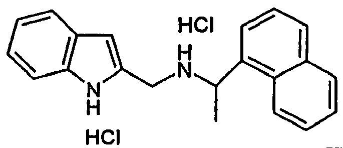 | 69 ± 9 |
| **PHD 337-R** | C₂₁H₂₀N₂.2HCl 373,32 | 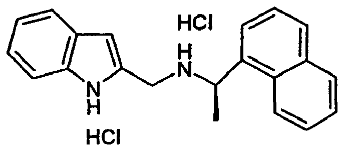 | 73 ± 10 |
| **PHD 337-S** | C₂₁H₂₀N₂.2HCl 373,32 | 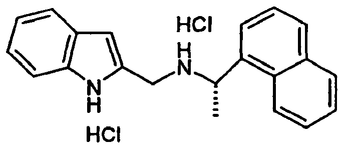 | 3 ± 2 |
| **PHD 356** | C₂₉H₂₀N₂O.2HCl 353,29 | 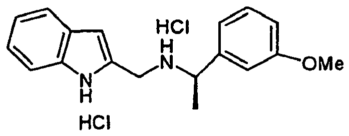 | 76 ± 1 |
| **PHD 363** | C₂₁H₁₉ClN₂.2HCl 407,77 | 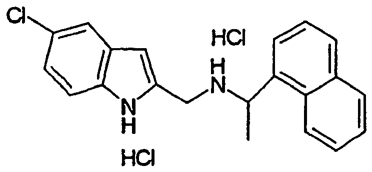 | 6 ± 3 |

### 4.2 Spécificité de l'activité de ces molécules

Les molécules PHD, utilisées à une concentration de 10 µM ne conduisent pas ou peu à l'accumulation d'[³H]IP dans des cellules CHO(WT*) témoins ce qui suggère leur spécificité d'action vis-à-vis du CaSR (Tableau 3). Les cellules CHO(WT*) ont été tranfectées avec le plasmide seul et n'expriment pas le CaSR

L'accumulation d'inositols phosphates tritiés est exprimée en pourcentage du taux de base observé en présence de 2 mM Ca²⁺ (100 %) dans les cellules CHO(WT*) ou CHO(CaSR).

**Tableau 3: Accumulation d'inositols phosphates tritiés dans les cellules CHO(CaSR) et CHO(WT*) induite par les composés PHD/AK et le composé calcimimétique NPS R-568**

| Composé - (10 µM) | Structure | Accumulation d'(³H)-IP % du taux basal. | |
|---|---|---|---|
| | | CHO (WT*) | CHO (CaSR) |
| NPS R-568 | 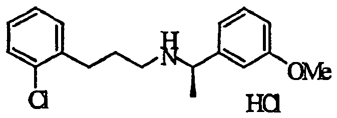 | 110 ± 9 | 454 ± 33 |
| PHD 337 | 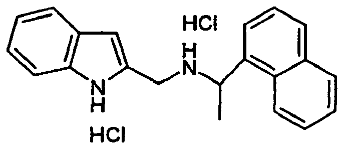 | 147 ± 14 | 444 ± 101 |
| PHD 337-R | 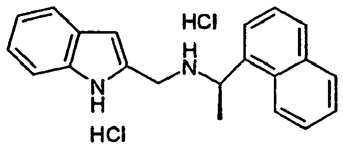 | 130 ± 9 | 588 ± 129 |
| PHD 337-S | 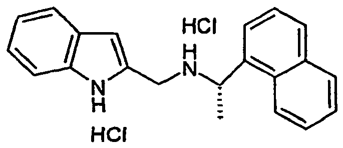 | 132 ± 1 | 225 ± 7 |
| PHD 356 | 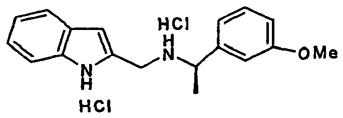 | 151 ± 7 | 482 ± 58 |
| PHD 359 | 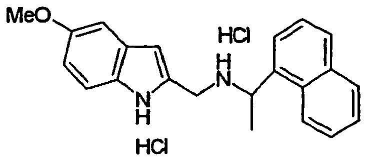 | 117 ± 8 | 303 ± 42 |
| PHD 361 | 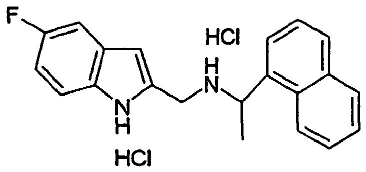 | 133 ± 3 | 420 ± 70 |
| PHD 363 | 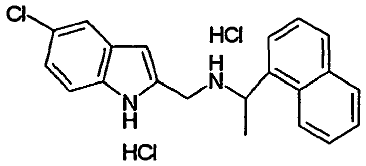 | 125 ± 12 | 325 ± 67 |
| PHD 368 | 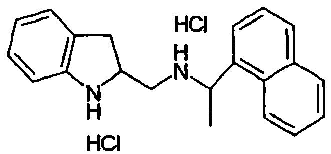 | 126 ± 6 | 388 ± 85 |
| PHD 338 | 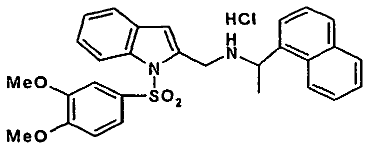 | 111 ± 10 | 202 ± 9 |
| AK 43 | 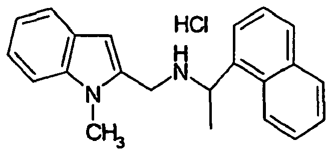 | 109 ± 2 | 401 ± 16 |

## Revendications

1. Composé de formule générale II suivante : 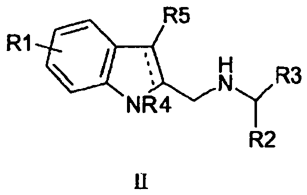 dans laquelle :
le groupe R1 représente un atome d'hydrogène ou d'halogène ou un groupe alkyle ou alkoxy en C₁-C₆,
le groupe R5 représente un groupe alkyle en C₁-C₆ ou un atome d'hydrogène ou un atome d'halogène,
le groupe R2 représente un atome d'hydrogène ou un groupe alkyle en C₁-C₆,
le groupe R3 représente un groupe aryle
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur,
à l'exception des composés dont les formules sont les suivantes ; 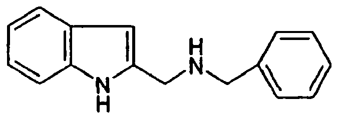 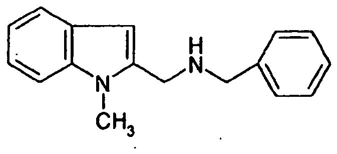 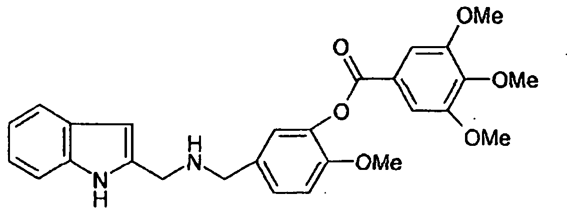 et

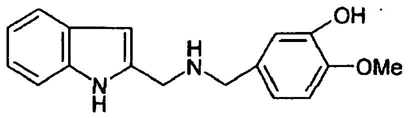

2. Composé selon la revendication 1, **caractérisé en ce qu'**il est représenté par la formule III suivante : 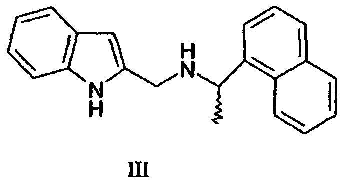 et son sel avec un acide pharmaceutiquement acceptable, sous forme d'un mélange racémique ou de son isomère optiquement pur.

3. Composé selon la revendication 1, **caractérisé en ce qu'**il est représenté par la formule IV suivante : 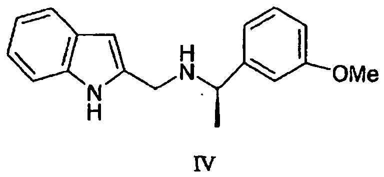 et son sel avec un acide pharmaceutiquement acceptable.

4. Procédé de préparation du composé de formule générale II suivante : 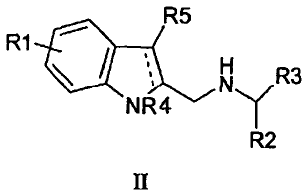 dans laquelle :
le groupe R1 représente un atome d'hydrogène ou d'halogène ou un groupe alkyle ou alkoxy en C₁-C₆,
le groupe R5 représente un groupe alkyle en C₁-C₆ ou un atome d'hydrogène ou un atome d'halogène,
le groupe R2 représente un atome d'hydrogène ou un groupe alkyle en C₁-C₆,
le groupe R3 représente un groupe aryle
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur
- à l'exception des composés de formules 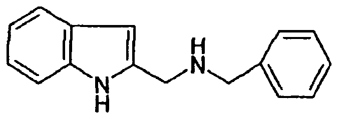 et 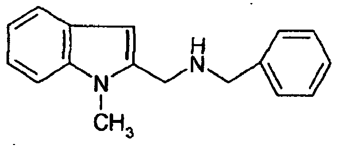 **caractérisé en ce qu'**il comporte l'étape de réduction de l'amide de formule générale V suivante: 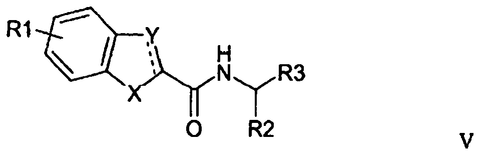 dans laquelle :
le groupe X représente un groupe -NR4,
le groupe Y représente un groupe -CR5 ou -CHR5,
le groupe R1 représente un atome d'hydrogène ou d'halogène ou un groupe alkyle ou alkoxy en C₁-C₆,
le groupe R5 représente un atome d'hydrogène ou un groupe alkyle en C₁-C₆ ou un atome d'halogène,
le groupe R2 représente un atome d'hydrogène ou un groupe alkyle en C₁-C₆,
le groupe R3 représente un groupe aryle
et le groupe R4 représente un atome d'hydrogène, un groupe alkyle, aryle, aralkyle, alkylsulfonamide, arylsulfonamide ou aralkylsulfonamide
à l'exception des composés de formule

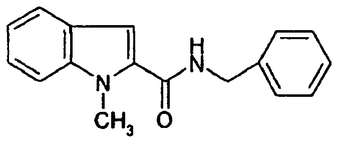

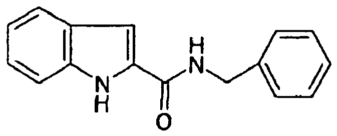

5. Procédé selon la revendication 4, **caractérisé en ce qu'**il comporte l'étape préalable de réaction entre un chlorure d'acyle de formule générale VI suivante : 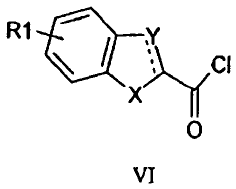 dans laquelle :
le groupe X représente un groupe -NR4,
le groupe Y représente un groupe -CR5 ou -CHR5,
le groupe R1 représente un atome d'hydrogène ou d'halogène ou un groupe alkyle ou alkoxy en C₁-C₆,
le groupe R5 représente un atome d'hydrogène ou un groupe alkyle en C₁-C₆ ou un atome d'halogène,
et le groupe R4 représente un atome d'hydrogène, un groupe alkyle, aryle, aralkyle, alkylsulfonamide, arylsulfonamide ou aralkylsulfonamide,
et une amine de formule générale VII suivante : 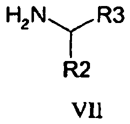 dans laquelle :
le groupe R2 représente un atome d'hydrogène ou un groupe alkyle en C₁-C₆
et le groupe R3 représente un groupe aryle
pour obtenir l'amide de formule générale V.

6. Procédé selon la revendication 5, **caractérisé en ce qu'**il comporte l'étape préalable de transformation de l'acide carboxylique de formule générale VIII suivante : 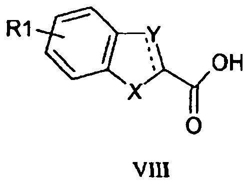 dans laquelle:
le groupe X représente un groupe -NR4,
le groupe Y représente un groupe -CR5 ou -CHR5,
le groupe R1 représente un atome d'hydrogène ou d'halogène ou un groupe alkyle ou alkoxy en C₁-C₆,
le groupe R5 représente un groupe alkyle en C₁-C₆ ou un atome d'hydrogène ou un atome d'halogène,
et le groupe R4 représente un atome d'hydrogène, un groupe alkyle, aryle, aralkyle, alkylsulfonamide, arylsulfonamide ou aralkylsulfonamide,
en chlorure d'acyle de formule générale VII, de préférence par réaction avec un chlorure de thionyle.

7. Amide de formule générale V suivante: 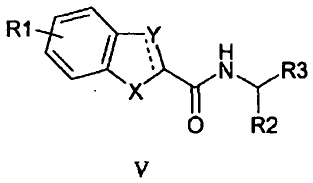 dans laquelle:
le groupe X représente un groupe -NR4,
le groupe Y représente un groupe -CR5 ou -CHR5,
le groupe R1 représente un atome d'hydrogène ou d'halogène ou un groupe alkyle ou alkoxy en C₁-C₆,
le groupe R5 représente un groupe alkyle en C₁-C₆ ou un atome d'hydrogène ou un atome d'halogène,
le groupe R2 représente un groupe alkyle en C₁-C₆ ou un atome d'hydrogène,
le groupe R3 représente un groupe aryle,
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur,
à l'exception des composés de formule :

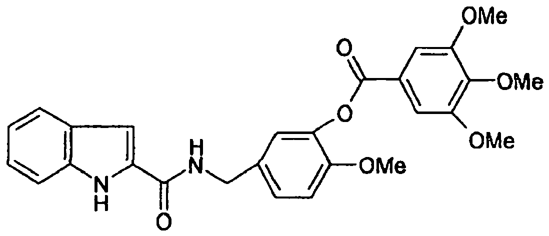

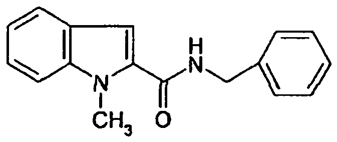

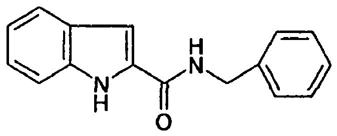

8. Composition pharmaceutique comprenant un composé de formule générale, II suivante : 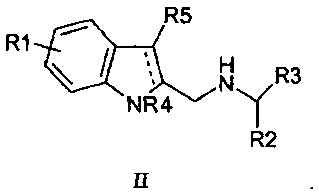 dans laquelle :
le groupe R1 représente un atome d'hydrogène ou d'halogène ou un groupe alkyle ou alkoxy en C₁-C₆,
le groupe R5 représente un groupe alkyle en C₁-C₆ ou un atome d'hydrogène ou un atome d'halogène,
le groupe R2 représente un atome d'hydrogène ou un groupe alkyle en C₁-C₆,
le groupe R3 représente un groupe aryle
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur,
ou un composé selon l'une quelconque des revendications 2 ou 3 prise séparément et un support pharmaceutique approprié.

9. Composés de formule générale II suivante : 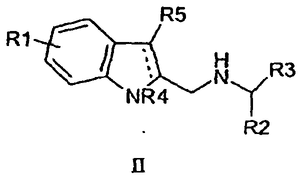 dans laquelle :
le groupe R1 représente un atome d'hydrogéne ou d'halogène ou un groupe alkyle ou alkoxy en C₁-C₆,
le groupe R5 représente un groupe alkyle en C₁-C₆ ou un atome d'hydrogène ou un atome d'halogène,
le groupe R2 représente un atome d'hydrogène ou un groupe alkyle en C₁-C₆,
te groupe R3 représente un groupe aryle
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur,
composés selon l'une quelconque des revendications 2 ou 3, ou composition selon la revendication 8 pour l'utilisation comme modulateur de l'activité du CaSR.

10. Composés pour l'utilisation selon la revendication 9, **caractérisée en ce que** le CaSR se trouve dans la glande parathyroïde, la thyroïde, les cellules osseuses, l'estomac, le poumon, le rein, l'hypophyse, le cerveau, l'hypothalamus, les aires olfactives ou l'hippocampe

11. Composés pour l'utilisation selon l'une quelconque des revendications 9 et 10 prise séparément, **caractérisée en ce que** ces composés sont plus sélectifs vis à vis des récepteurs de la parathyroïde comparativement à ceux de la glande thyroïde.

12. Composé de formule générale II suivante : 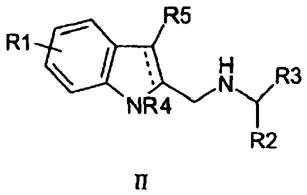 dans laquelle :
le groupe R1 représente un atome d'hydrogène ou d'halogène ou un groupe alkyle ou alkoxy en C₁-C₆,
le groupe R5 représente un groupe alkyle en C₁-C₆ ou un atome d'hydrogène ou un atome d'halogène,
le groupe R2 représente un atome d'hydrogène ou un groupe alkyle en C₁-C₆,
le groupe R3 représente un groupe aryle
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur,
composés selon l'une quelconque des revendications 2 ou 3 ou composition selon la revendication 8 pour son utilisation comme médicament.

13. Utilisation des composés de formule générale II suivante : 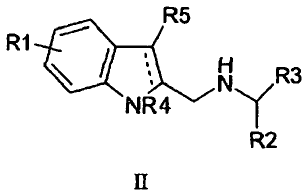 dans laquelle :
le groupe R1 représente un atome d'hydrogène ou d'halogène ou un groupe alkyle ou alkoxy en C₁-C₆,
le groupe R5 représente un groupe alkyle en C1-C6 ou un atome d'hydrogène ou un atome d'halogène,
le groupe R2 représente un atome d'hydrogène ou un groupe alkyle en C₁-C₆,
le groupe R3 représente un groupe aryle
et le groupe R4 représente un atome d'hydrogène, un groupe alkyle, aryle, aralkyle, alkylsulfonamide, arylsulfonamide ou aralkylsulfonamide,
et son sel avec un acide pharmaceutiquement acceptable, sous forme d'un mélange racémique ou de son isomère optiquement pur,
des composés selon l'une quelconque des revendications 2 ou 3 et de la composition selon la revendication 8 pour la fabrication d'un médicament destiné au traitement des maladies ou des désordres physiologiques liés à des perturbations de l'activité des CaSR.

14. Utilisation selon la revendication 13 **caractérisée en ce que** les maladies ou les désordres physiologiques sont du type hyperparathyroïdies primaires ou secondaires, ostéoporose, maladies cardio-vasculaires, gastro-intestinales, endocrines, neurodégénératives ou certains cancers où les ions (Ca²⁺)_{c} sont anormalement élevés.

15. Utilisation selon la revendication 14 **caractérisée en ce que** l'hyperparathyroïdie secondaire est observée lors d'insuffisance rénale chronique.

## Claims

1. Compound having the following general formula II: 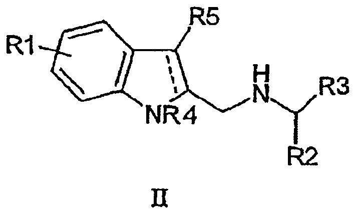 in which:
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a hydrogen atom or a C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
with the exception of the compounds which have the following formulae; 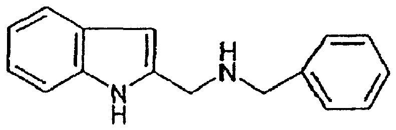 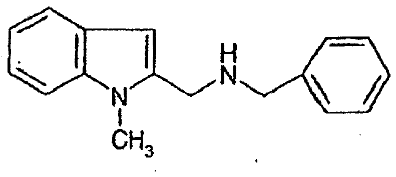 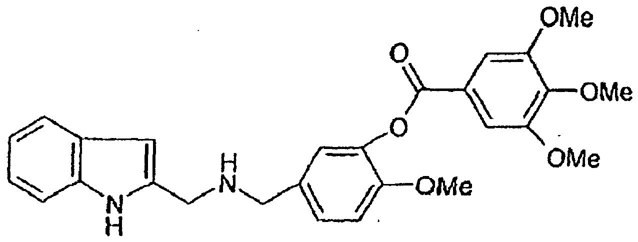 and

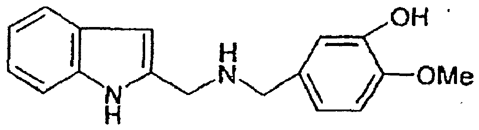

2. Compound as claimed in claim 1, **characterised in that** it is represented by the following formula III: 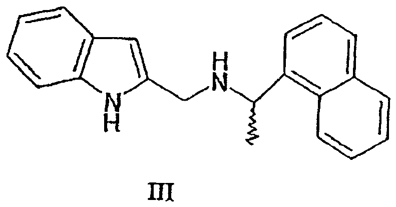 and the salt thereof with a pharmaceutically acceptable acid, in the form of a racemic mixture or of the optically pure isomer thereof.

3. Compound as claimed in claim 1, **characterised in that** it is represented by the following formula IV: 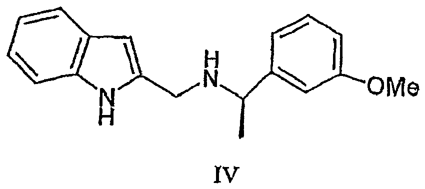 and the salt thereof with a pharmaceutically acceptable acid.

4. Method for preparing the compound having the following general formula II: 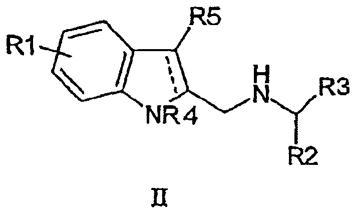 in which:
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a hydrogen atom or C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
- with the exception of the compounds which have the following formulae 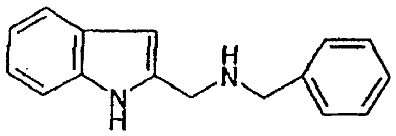 and 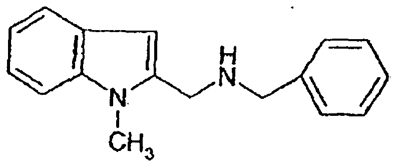
**characterised in that** it comprises the step for reducing the amide having the following general formula V: 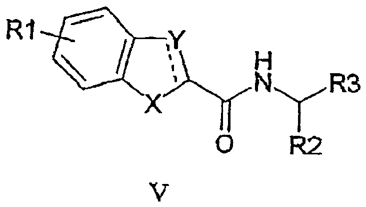 in which:
the group X represents a group -NR4,
the group Y represents a group -CR5 or -CHR5,
the group R1 represents a hydrogen or halogen atom or a C₁-C₆ alkyl or alkoxy group,
the group R5 represents a hydrogen atom or a C₁-C₆ alkyl group or a halogen atom,
the group R2 represents a hydrogen atom or a C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
with the exception of the compounds which have the following formulae

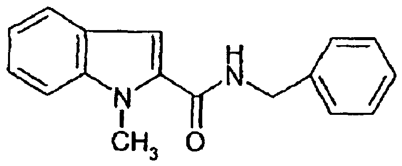

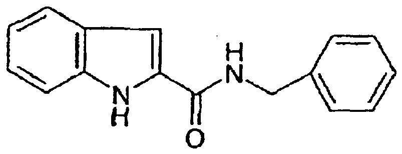

5. Method as claimed in claim 4, **characterised in that** it comprises the prior reaction step between an acyl chloride having the following general formula VI: 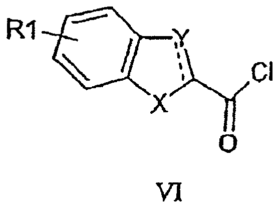 in which:
the group X represents a group -NR4,
the group Y represents a group -CR5 or -CHR5,
the group R1 represents a hydrogen or halogen atom or a C₁-C₆ alkyl or alkoxy group,
the group R5 represents a hydrogen atom or a C₁-C₆ alkyl group or a halogen atom,
and the group R4 represents a hydrogen atom, an alkyl, aryl, aralkyl, alkylsulphnamide, arylsulphonamide or aralkylsulphonamide group,
and an amine having the following general formula VII: 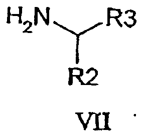 in which:
the group R2 represents a hydrogen atom or a C₁-C₆ alkyl group,
the group R3 represents an aryl group so as to obtain the amide having the general formula V.

6. Method as claimed in claim 5, **characterised in that** it comprises the prior step for converting the carboxylic acid having the following general formula VIII: 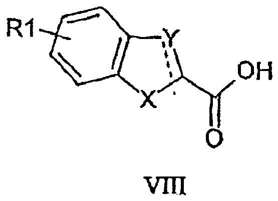 in which:
the group X represents a group -NR4,
the group Y represents a group -CR5 or -CHR5,
the group R1 represents a hydrogen or halogen atom or a C₁-C₆ alkyl or alkoxy group,
the group R5 represents a C₁-C₆ alkyl group or a hydrogen atom or a halogen atom,
and the group R4 represents a hydrogen atom, an alkyl, aryl, aralkyl, alkylsulphnamide, arylsulphonamide or aralkylsulphonamide group, into acyl chloride having the general formula VII, preferably by reacting with a thionyl chloride.

7. Amide having the following general formula V: 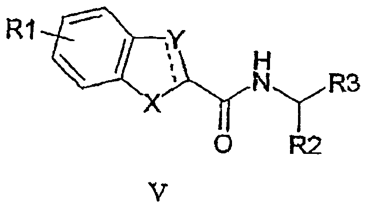 in which:
the group X represents a group -NR4,
the group Y represents a group -CR5 or -CHR5,
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a C₁-C₆ alkyl group or a hydrogen atom,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
with the exception of the compounds which have the following formulae:

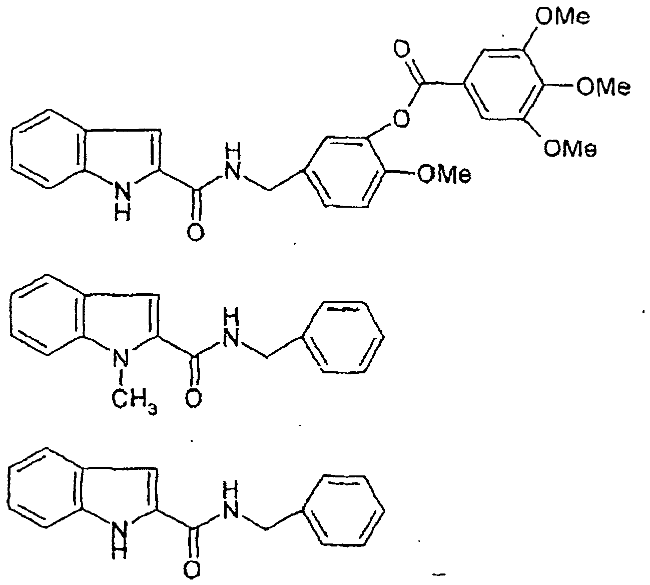

8. Pharmaceutical composition comprising a compound having the following general formula II: 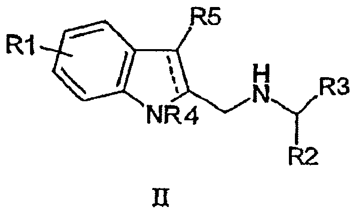 in which:
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a hydrogen atom or a C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
or a compound as claimed in any one of claims 2 or 3 taken separately, and a suitable pharmaceutical carrier.

9. Compounds having the following general formula II: 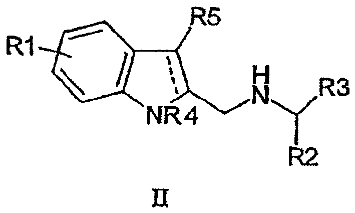 in which:
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a hydrogen atom or a C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
compounds as claimed in any one of claims 2 or 3, or composition as claimed in claim 8 for use as a modulator of CaSR activity.

10. Compounds for the use as claimed in claim 9, **characterised in that** the CaSR is found in the parathyroid gland, the thyroid, bone cells, the stomach, the lung, the kidney, the pituitary gland, the brain, the hypothalamus, the olfactory areas or the hippocampus.

11. Compounds for the use as claimed in any one of claims 9 and 10 taken separately, **characterised in that** these compounds are more selective with respect to the receptors of the parathyroid compared with those of the thyroid gland.

12. Compound having the following general formula II: 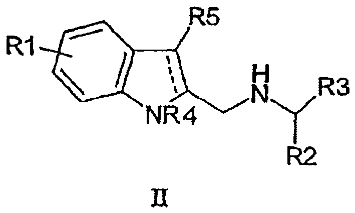 in which:
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a hydrogen atom or a C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
compounds as claimed in any one of claims 2 or 3, or composition as claimed in claim 8 for the use thereof as a medicinal product.

13. Use of the compounds having the following general formula II: 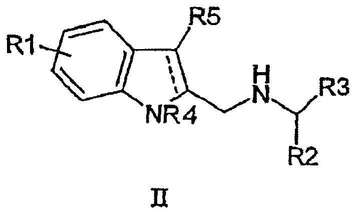 in which:
the group R1 represents a hydrogen or halogen atom or a C₁-C₆ alkyl or alkoxy group,
the group R5 represents a C₁-C₆ alkyl group or a hydrogen atom or a halogen atom,
the group R2 represents a hydrogen atom or a C₁-C₆ alkyl group,
the group R3 represents an aryl group,
and the group R4 represents a hydrogen atom, an alkyl, aryl, aralkyl, alkylsulphonamide, arylsulphonamide or aralkylsulphonamide group,
and the salt thereof with a pharmaceutically acceptable acid, in the form of a racemic mixture or of the optically pure isomer thereof,
and of the compounds as claimed in any one of claims 2 or 3, and of the composition as claimed in claim 8 for producing a medicinal product intended for the treatment of physiological disorders and diseases associated with disturbances of CaSR activity.

14. Use as claimed in claim 13, **characterised in that** the physiological disorders or diseases are of the type including primary or secondary hyperparathyroidism, osteoporosis, cardiovascular, gastrointestinal, endocrine or neurodegenerative diseases, or certain cancers in which (Ca²⁺)ₑ ions are abnormally high.

15. Use as claimed in claim 14, **characterised in that** the secondary hyperparathyroidism is observed in chronic renal failure.

## Patentansprüche

1. Verbindung der folgenden allgemeinen Formel II: 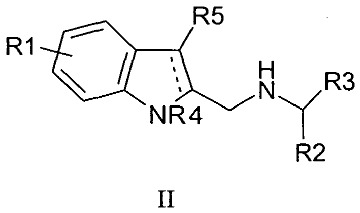 in welcher:
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 ein Wasserstoffatom oder eine C₁ bis C₆-Alkylgruppe darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
mit Ausnahme der Verbindungen mit den folgenden Formeln: 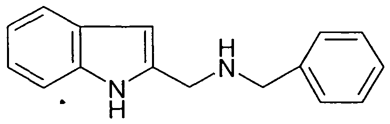 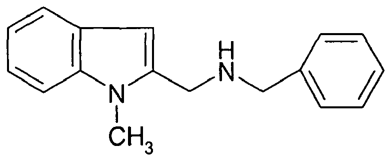 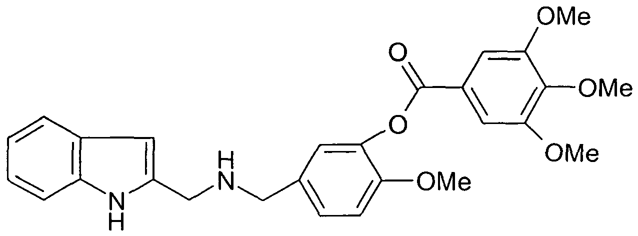 und

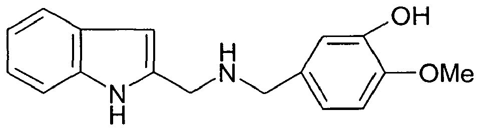

2. Verbindung nach Anspruch 1, **dadurch gekennzeichnet, dass** sie durch die folgende Formel III wiedergegeben ist: 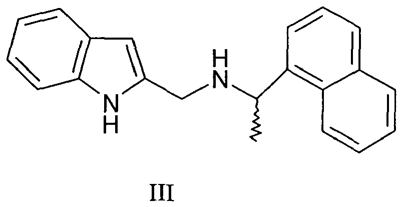 und deren Salz mit einer pharmazeutisch verträglichen Säure, in Form eines racemischen Gemisches oder deren optisch reinen Isomers.

3. Verbindung nach Anspruch 1, **dadurch gekennzeichnet, dass** sie durch die folgende Formel IV wiedergegeben ist: 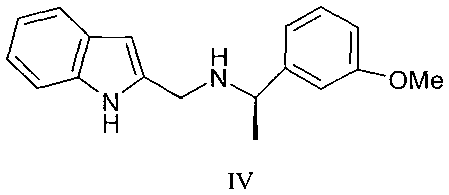 und deren Salz mit einer pharmazeutisch verträglichen Säure.

4. Verfahren zur Herstellung der Verbindung der folgenden allgemeinen Formel II: 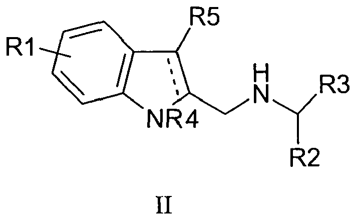 in welcher:
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 ein Wasserstoffatom oder eine C₁ bis C₆-Alkylgruppe darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
- mit Ausnahme der Verbindungen der Formeln 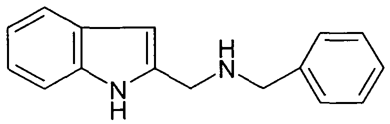 und 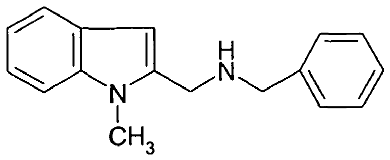 **dadurch gekennzeichnet, dass** es den Schritt der Reduktion des Amids der folgenden allgemeinen Formel V umfasst: 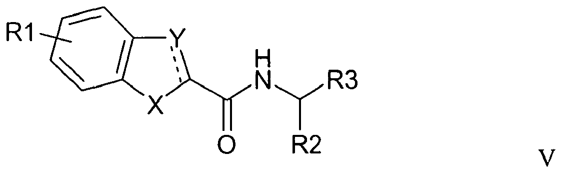
in welcher:
die Gruppe X eine Gruppe -NR4 darstellt,
die Gruppe Y eine Gruppe -CR5 oder -CHR5 darstellt,
die Gruppe R1 ein Wasserstoffatom oder Halogenatom oder eine C₁ bis C₆-Alkyl- oder -Alkoxygruppe darstellt,
die Gruppe R5 ein Wasserstoffatom oder eine C₁ bis C₆-Alkylgruppe oder ein Halogenatom darstellt,
die Gruppe R2 ein Wasserstoffatom oder eine C₁ bis C₆-Alkylgruppe darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt
mit Ausnahme der Verbindungen der Formeln

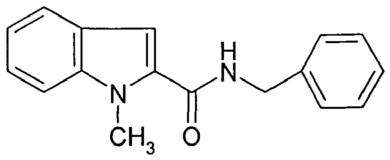

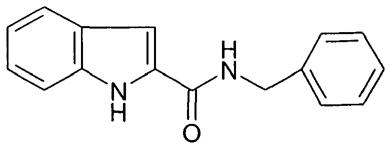

5. Verfahren nach Anspruch 4, **dadurch gekennzeichnet, dass** es einen vorherigen Schritt der Reaktion umfasst zwischen einem Acylchlorid der folgenden allgemeinen Formel IV: 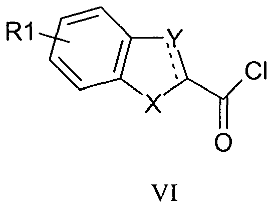 in welcher:
die Gruppe X eine Gruppe -NR4 darstellt,
die Gruppe Y eine Gruppe -CR5 oder -CHR5 darstellt,
die Gruppe R1 ein Wasserstoffatom oder Halogenatom oder eine C₁ bis C₆-Alkyl- oder -Alkoxygruppe darstellt,
die Gruppe R5 ein Wasserstoffatom oder eine C₁ bis C₆-Alkylgruppe oder ein Halogenatom darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und einem Amin der folgenden allgemeinen Formel VII: 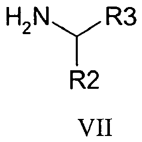 in welcher:
die Gruppe R2 ein Wasserstoffatom oder eine C₁ bis C₆-Alkylgruppe darstellt,
die Gruppe R3 eine Arylgruppe darstellt,
um das Amid der allgemeinen Formel V zu erhalten.

6. Verfahren nach Anspruch 5, **dadurch gekennzeichnet, dass** es den vorherigen Schritt der Umwandlung umfasst der Carboxylsäure der folgenden allgemeinen Formel VIII: 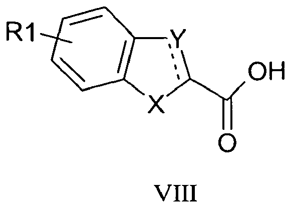 in welcher:
die Gruppe X eine Gruppe -NR4 darstellt,
die Gruppe Y eine Gruppe -CR5 oder -CHR5 darstellt,
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
in Acylchlorid der allgemeinen Formel VII, vorzugsweise durch Reaktion mit einem Thionylchlorid.

7. Amid der folgenden allgemeinen Formel V: 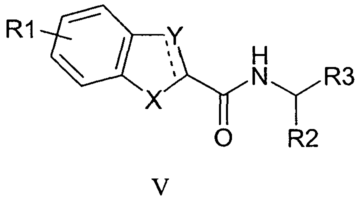 in welcher:
die Gruppe X eine Gruppe -NR4 darstellt,
die Gruppe Y eine Gruppe -CR5 oder -CHR5 darstellt,
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 eine C₁ bis C₆-Alkylgruppe oder ein Wasserstoffatom darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
mit Ausnahme der Verbindungen der Formeln:

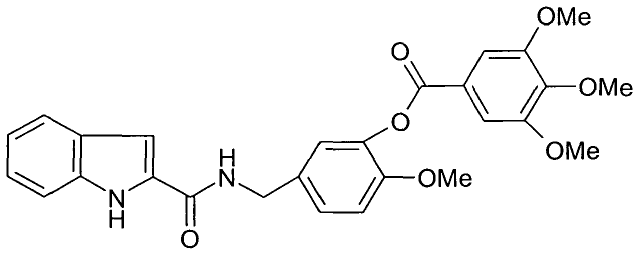

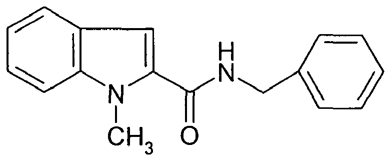

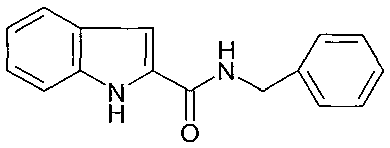

8. Pharmazeutische Zusammensetzung, enthaltend eine Verbindung der folgenden allgemeinen Formel II: 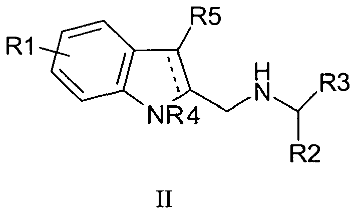 in welcher:
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 ein Wasserstoffatom oder eine C₁ bis C₆-Alkylgruppe darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
oder eine Verbindung nach einem der Ansprüche 2 oder 3, einzeln genommen, und einen geeigneten pharmazeutischen Träger.

9. Verbindungen der folgenden allgemeinen Formel II: 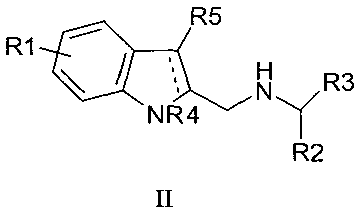 in welcher:
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 ein Wasserstoffatom oder eine C₁ bis C₆-Alkylgruppe darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
Verbindungen nach einem der Ansprüche 2 oder 3 oder Zusammensetzung nach Anspruch 8 für die Verwendung als Modulator der Aktivität des CaSR.

10. Verbindungen für die Verwendung nach Anspruch 9, **dadurch gekennzeichnet, dass** sich das CaSR in der Nebenschilddrüse, der Schilddrüse, den Knochenzellen, dem Magen, der Lunge, der Niere, der Hypophyse, dem Gehirn, dem Hypothalamus, den olfaktorischen Bereichen oder dem Hippocampus befindet.

11. Verbindungen für die Verwendung nach einem der Ansprüche 9 und 10, einzeln genommen, **dadurch gekennzeichnet, dass** diese Verbindungen gegenüber Rezeptoren der Nebenschilddrüse im Vergleich zu denen der Schilddrüse selektiver sind.

12. Verbindung der folgenden allgemeinen Formel II: 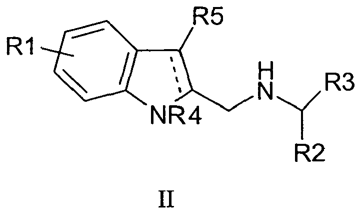 in welcher:
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 ein Wasserstoffatom oder eine C₁ bis C₆-Alkylgruppe darstellt,
die Gruppe R3 eine Arylgruppe darstellt
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
Verbindungen nach einem der Ansprüche 2 oder 3 oder Zusammensetzung nach Anspruch 8 für ihre Verwendung als Arzneimittel.

13. Verwendung der Verbindungen der folgenden allgemeinen Formel II: 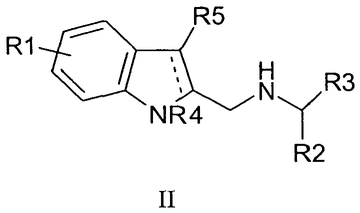 in welcher:
die Gruppe R1 ein Wasserstoffatom oder Halogenatom oder eine C₁ bis C₆-Alkyl- oder -Alkoxygruppe darstellt,
die Gruppe R5 eine C₁ bis C₆-Alkylgruppe oder ein Wasserstoffatom oder ein Halogenatom darstellt,
die Gruppe R2 ein Wasserstoffatom oder eine C₁ bis C₆-Alkylgruppe darstellt,
die Gruppe R3 eine Arylgruppe darstellt,
und die Gruppe R4 ein Wasserstoffatom, eine Alkylgruppe, Arylgruppe, Aralkylgruppe, Alkylsulfonamidgruppe, Arylsulfonamidgruppe oder Aralkylulfonamidgruppe darstellt,
und deren Salz mit einer pharmazeutisch verträglichen Säure, in Form eines racemischen Gemisches oder deren optisch reinen Isomers,
Verbindungen nach einem der Ansprüche 2 oder 3 und Zusammensetzung nach Anspruch 8 für die Herstellung eines Arzneimittels, das für die Behandlung von Erkrankungen oder physiologischen Störungen, die mit Störungen der CaSR-Aktivität verbunden sind, bestimmt ist.

14. Verwendung nach Anspruch 13, **dadurch gekennzeichnet, dass** die Erkrankungen oder physiologischen Störungen vom Typ primäre oder sekundäre Hyperparathyreoidismen, Osteoporose, Herz-Kreislauf-Erkrankungen, Magen-DarmErkrankungen, endokrine Erkrankungen, neurodegenerative Erkrankungen oder bestimmter Krebsarten, wo die (Ca²⁺)ₑ-Ionen abnormal erhöht sind, sind.

15. Verwendung nach Anspruch 14, **dadurch gekennzeichnet, dass** der sekundäre Hyperparathyreoidismus während einer chronischen Niereninsuffizienz beobachtet wird.
